(19)

Europäisches
Patentamt
European
Patent Office
Office européen
des brevets

(11) **EP 3 834 736 A1**

(12) **EUROPEAN PATENT APPLICATION**
published in accordance with Art. 153(4) EPC

(43) Date of publication:
**16.06.2021 Bulletin 2021/24**

(21) Application number: **19937359.8**

(22) Date of filing: **31.12.2019**

(51) Int Cl.:
***A61B 8/08*** (2006.01)

(86) International application number:
**PCT/CN2019/130274**

(87) International publication number:
**WO 2021/082272 (06.05.2021 Gazette 2021/18)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME
KH MA MD TN**

(30) Priority: **31.10.2019 CN 201911054151**

(71) Applicant: CHISON Medical Technologies Co., Ltd.
**Wuxi, Jiangsu 214028 (CN)**

(72) Inventors:
• **SHAO, Renjie
  Wuxi
  Jiangsu 214028 (CN)**
• **ZHAO, Mingchang
  Wuxi
  Jiangsu 214028 (CN)**
• **GAN, Conggui
  Wuxi
  Jiangsu 214028 (CN)**

(74) Representative: **Finnegan Europe LLP
1 London Bridge
London SE1 9BG (GB)**

(54) **METHOD FOR DETERMINING CARDIAC CYCLES AND ULTRASONIC DEVICE**

(57) The present invention relates to the technical field of image processing, in particular to a method for determining a cardiac cycle and ultrasonic equipment. The method comprises: acquiring a cardiac ultrasound video; classifying the cardiac ultrasound video by using a section type recognition model to determine a section type of the cardiac ultrasound video; and processing the cardiac ultrasound video by using a systole and diastole recognition model corresponding to the section type to obtain the cardiac cycle corresponding to the cardiac ultrasound video. The model is used to process the cardiac ultrasound video to detect the corresponding cardiac cycle. Model detection can avoid the use of an electrocardiograph and simplify the detection of the cardiac cycle. Furthermore, real-time detection of the cardiac cycle can be realized during echocardiography.

EP 3 834 736 A1

```
                                                          ┌──────────────┐ S11
              ┌─────────────────────────────────────┐───┘
              │   acquiring a cardiac ultrasound video   │
              └─────────────────────────────────────┘
                              │
              ┌─────────────────────────────────────┐───┐ S12
              │  classifying the cardiac ultrasound video by │
              │  using a section type recognition model to   │
              │   determine a section type of the cardiac    │
              │          ultrasound video                    │
              └─────────────────────────────────────┘
                              │
              ┌─────────────────────────────────────┐───┐ S13
              │  processing the cardiac ultrasound video by  │
              │  using a systole and diastole recognition model │
              │  corresponding to the section type to obtain the │
              │   cardiac cycle corresponding to the cardiac  │
              │          ultrasound video                    │
              └─────────────────────────────────────┘
```

Fig. 1

## Description

### Field

The present invention relates to the technical field of image processing, in particular to a method for determining a cardiac cycle and ultrasonic equipment.

### Background

The mechanical activity cycle formed by every contraction and relaxation of the heart is called cardiac cycle, and the cardiac cycles of both atriums and ventricles include systole and diastole.
During cardiac cycle detection, an electrocardiograph is usually used to record the electrical activity change pattern produced by each cardiac cycle of the heart from the body surface, and the cardiac cycle is determined from the electrical activity change pattern. However, this method for detecting the cardiac cycle needs to connect an object to be detected with the electrocardiograph, which makes the detection process complicated.

### Summary

In view of this, the embodiments of the present invention provide a method for determining a cardiac cycle and ultrasonic equipment to solve the problem that the cardiac cycle determination process is complicated.
According to a first aspect, an embodiment of the present invention provides a method for determining a cardiac cycle, comprising:

acquiring a cardiac ultrasound video;
classifying the cardiac ultrasound video by using a section type recognition model to determine a section type of the cardiac ultrasound video; and
processing the cardiac ultrasound video by using a systole and diastole recognition model corresponding to the section type to obtain the cardiac cycle corresponding to the cardiac ultrasound video.

According to the method for determining the cardiac cycle provided by the embodiment of the present invention, the model is adopted to process the cardiac ultrasound video so as to detect the corresponding cardiac cycle. Model detection can avoid the use of an electrocardiograph and simplify the detection of the cardiac cycle. Furthermore, real-time detection of the cardiac cycle can be realized during echocardiography.
Based on the first aspect, in a first implementation mode of the first aspect, a segmentation model corresponding to the section type is adopted to conduct cardiac chamber segmentation of the cardiac ultrasound video to obtain pixels of a cardiac chamber; and
cardiac parameters corresponding to the cardiac ultrasound video are determined at least according to the pixels of the cardiac chamber and the cardiac cycle, wherein the cardiac parameters include at least one of ejection fraction, end-diastolic volume, end-systolic volume and target cardiac chamber weight.
According to the method for determining the cardiac cycle provided by the embodiment of the present invention, the pixels of each cardiac chamber can be obtained by classification of the cardiac ultrasound video and segmentation of the cardiac chamber, and then the cardiac parameters corresponding to the cardiac ultrasound video are determined based on the pixels of each cardiac chamber; that is to say, by processing the cardiac ultrasound video with the model, the accuracy of segmentation of each cardiac chamber in each frame of image of the cardiac ultrasound video is improved, and thus the accuracy of calculation of the cardiac parameters can be improved.
Based on the first aspect or the first implementation mode of the first aspect, in a second implementation mode of the first aspect,
the step of processing the cardiac ultrasound video by using the systole and diastole recognition model corresponding to the section type to obtain the cardiac cycle corresponding to the cardiac ultrasound video comprises:

acquiring feature information of each frame of image in the cardiac ultrasound video by using the systole and diastole recognition model corresponding to the section type; and
determining an end systole and/or an end diastole in the cardiac ultrasound video according to the feature information to obtain the cardiac cycle corresponding to the cardiac ultrasound video.

According to the method for determining the cardiac cycle provided by the embodiment of the present invention, because different section types correspond to different systoles and diastoles, by acquiring the feature information of each frame of image in the cardiac ultrasound video by using the systole and diastole recognition model corresponding to the section type, the accuracy of acquiring the feature information can be improved.

Based on the second implementation mode of the first aspect, in a third implementation mode of the first aspect, the feature information of each frame of image in the cardiac ultrasound video is represented by a preset identifier, wherein a first preset identifier corresponds to the systole and a second preset identifier corresponds to the diastole; and the step of determining the end systole and/or the end diastole in the cardiac ultrasound video according to the feature information to obtain the cardiac cycle corresponding to the cardiac ultrasound video comprises:

traversing the preset identifier corresponding to each frame of image, determining an image frame corresponding to the first preset identifier when the preset identifier experiences a change from the first preset identifier to the second preset identifier as a first image frame, and/or determining an image frame corresponding to the second preset identifier when the preset identifier experiences a change from the second preset identifier to the first preset identifier as a second image frame, wherein the first image frame corresponds to the end systole and the second image frame corresponds to the end diastole; and

detecting the cardiac cycle corresponding to the cardiac ultrasound video based on the first image frame and/or the second image frame.

Based on the second implementation mode of the first aspect, in a fourth implementation mode of the first aspect, the feature information of each frame of image in the cardiac ultrasound video is represented by a coefficient, the coefficient is used to indicate the size of a target cardiac chamber in systole and diastole, the coefficient increases progressively in the diastole of the cardiac cycle and the coefficient decreases progressively in the systole of the cardiac cycle; and

the step of determining the end systole and the end diastole according to the detected systole and diastole to detect the cardiac cycle corresponding to the cardiac ultrasound video comprises:

detecting the change trend of the coefficient to determine a third image frame corresponding to the end systole and/or a fourth image frame corresponding to the end diastole; and

detecting the cardiac cycle corresponding to the cardiac ultrasound video based on the third image frame and/or the fourth image frame.

According to the method for determining the cardiac cycle provided by the embodiment of the present invention, the coefficient is adopted to represent the feature information of each frame of image in the cardiac ultrasound video, so that the change process of the target cardiac chamber with time can be reflected, and the mechanical activity of the target cardiac chamber can be reflected more accurately, thereby improving the accuracy of determining the category of each frame of image.

Based on the second implementation mode of the first aspect, in a fifth implementation mode of the first aspect, the systole and diastole recognition model is trained in the following way:

acquiring a training set, wherein the training set comprises a sample cardiac ultrasound video and labeled data, the labeled data includes target feature information corresponding to each frame of image in the sample cardiac ultrasound video, the feature information is represented by a sample identifier or a sample coefficient, a first sample identifier corresponds to the systole and a second sample identifier corresponds to the diastole, and the sample coefficient is used for representing the size of the target cardiac chamber in systole and diastole;

inputting the sample cardiac ultrasound video into the systole and diastole recognition model to obtain predicted feature information corresponding to each frame of image in the sample cardiac ultrasound video; and

adjusting parameters of the systole and diastole recognition model based on the predicted feature information and the target feature information to train the systole and diastole recognition model.

According to the method for determining the cardiac cycle provided by the embodiment of the present invention, the sample coefficient is used for representing the size of the target cardiac chamber in systole and diastole, so that the change process of the target cardiac chamber with time can be reflected, and the mechanical activity of the target cardiac chamber can be reflected more accurately, thereby improving the classification accuracy of the systole and diastole recognition model obtained through training.

Based on the fifth implementation mode of the first aspect, in a sixth implementation mode of the first aspect, when the labeled data are expressed by the sample coefficient, the sample coefficient is calculated by the following method:

acquiring an electrocardiographic tracing corresponding to the sample cardiac ultrasound video; and

calculating the sample coefficient corresponding to each frame of image in the sample cardiac ultrasound video at least based on the electrocardiographic tracing.

Based on the sixth implementation mode of the first aspect, in a seventh implementation mode of the first aspect, the sample coefficient is calculated by the following formula:

$$y_t = \begin{cases} (\dfrac{\left|t - T_{ES}^i\right|}{\left|T_{ES}^i - T_{ED}^i\right|})^3, \; if \; T_{ED}^i < t \le T_{ES}^i \\[4mm] (\dfrac{\left|t - T_{ES}^i\right|}{\left|T_{ES}^i - T_{ED}^{i+1}\right|})^{1/3}, \; if \; T_{ES}^i < t \le T_{ED}^i \end{cases};$$

wherein i is the i-th cardiac cycle in the sample cardiac ultrasound video; t is the t-th frame of image in the sample cardiac ultrasound video; $T_{ED}^i$ is the time of end diastole in the i-th cardiac cycle; $T_{ES}^i$ is the time of end systole in the i-th cardiac cycle; and $y_t$ is the sample coefficient corresponding to the t-th frame of image in the sample cardiac ultrasound video.

Based on the first implementation mode of the first aspect, in an eighth implementation mode of the first aspect, the step of determining the cardiac parameters corresponding to the cardiac ultrasound video at least according to the pixels of the cardiac chamber and the cardiac cycle comprises:

determining a fifth image frame corresponding to the end systole and a sixth image frame corresponding to the end diastole in the cardiac ultrasound video based on the cardiac cycle;

extracting a target cardiac chamber obtained by segmentation from the adjacent fifth image frame and sixth image frame; and

calculating the cardiac parameters based on the pixels corresponding to the target cardiac chamber in the fifth image frame and the pixels corresponding to the target cardiac chamber in the sixth image frame.

Based on the eighth implementation mode of the first aspect, in a ninth implementation mode of the first aspect, the step of calculating the cardiac parameters based on the pixels corresponding to the target cardiac chamber in the fifth image frame and the pixels corresponding to the target cardiac chamber in the sixth image frame comprises:

counting the number of the pixels corresponding to the target cardiac chamber in the fifth image frame and the number of the pixels corresponding to the target cardiac chamber in the sixth image frame;

calculating the ejection fraction by using the counted numbers of the pixels;

or,

determining an end-diastolic area of the target cardiac chamber and an end-systolic area of the target cardiac chamber by using the counted numbers of the pixels;

performing linear fitting on the pixels corresponding to the target cardiac chamber in the fifth image frame and the pixels corresponding to the target cardiac chamber in the sixth image frame to determine a target cardiac chamber length corresponding to the end diastole and a target cardiac chamber length corresponding to the end systole; and

calculating an end-diastolic volume of the target cardiac chamber based on the end-diastolic area of the target cardiac chamber and the target cardiac chamber length corresponding to the end diastole; or calculating an end-systolic volume of the target cardiac chamber based on the end-systolic area of the target cardiac chamber and the target cardiac chamber length corresponding to the end systole.

Based on the ninth implementation mode of the first aspect, in a tenth implementation mode of the first aspect, the cardiac parameters are calculated by the following formula:

$$\text{ejection fraction} = \frac{Pix_{ED} - Pix_{ES}}{Pix_{ED}} * 100\% ;$$

wherein $Pix_{ED}$ is the number of the pixels corresponding to the target cardiac chamber in the sixth image frame, and $Pix_{ES}$ is the number of the pixels corresponding to the target cardiac chamber in the fifth image frame;

or,

$$\text{end-diastolic volume of target cardiac chamber} = 0.85 * S_{ED}^2 / L_{ED} ;$$

$$\text{end-systolic volume of target cardiac chamber} = \frac{0.85 * S_{ES}^{2}}{L_{ES}};$$

wherein $S_{ED}$ is the end-diastolic area of the target cardiac chamber, $L_{ED}$ is the target cardiac chamber length corresponding to the end diastole, $S_{ES}$ is the end-systolic area of the target cardiac chamber, and $L_{ES}$ is the target cardiac chamber length corresponding to the end systole.

Based on the first implementation mode of the first aspect, in an eleventh implementation mode of the first aspect, the step of determining the cardiac parameters corresponding to the cardiac ultrasound video at least according to the pixels of the cardiac chamber and the cardiac cycle comprises:

calculating a length and step size of sliding windows based on the cardiac cycle;

performing sliding window processing on the cardiac ultrasound video to obtain the number of pixels of each sliding window corresponding to the target cardiac chamber, so as to determine the number of pixels of the target cardiac chamber in the cardiac ultrasound video;

calculating an area of the target cardiac chamber and a length of the target cardiac chamber by using the number of the pixels of each sliding window corresponding to the target cardiac chamber;

acquiring a myocardial layer area corresponding to the target cardiac chamber, wherein the myocardial layer area corresponding to the target cardiac chamber is the product of the number of pixels of a myocardial layer corresponding to the target cardiac chamber in the cardiac ultrasound video and an area of each pixel; and

calculating a weight of the target cardiac chamber based on the area of the target cardiac chamber, the myocardial layer area corresponding to the target cardiac chamber and the length of the target cardiac chamber.

Based on the eleventh implementation mode of the first aspect, in a twelfth implementation mode of the first aspect, the step of performing sliding window processing on the cardiac ultrasound video to obtain the number of pixels of each sliding window corresponding to the target cardiac chamber, so as to determine the number of pixels of the target cardiac chamber in the cardiac ultrasound video comprises:

sliding on the cardiac ultrasound video based on the step size to determine target cardiac chambers included in each sliding window, wherein each sliding window comprises at least one image frame of the cardiac ultrasound video;

comparing the number of pixels of each target cardiac chamber included in each sliding window to obtain the number of pixels of each sliding window corresponding to the target cardiac chamber; and

taking the median of the number of pixels of each sliding window corresponding to the target cardiac chamber to obtain the number of the pixels of the target cardiac chamber in the cardiac ultrasound video.

Based on the twelfth implementation mode of the first aspect, in a thirteenth implementation mode of the first aspect, the weight of the target cardiac chamber is calculated by the following formula:

$$\text{weight of target cardiac chamber} =$$

$$1.05 * (\ (\tfrac{5}{6}) * (S_1 + S_2) * (L + t) - ((\tfrac{5}{6}) * S_2 * L);$$

$$t = \sqrt{\frac{(S_1 + S_2)}{\pi}} - \sqrt{\frac{S_2}{\pi}};$$

wherein $S_1$ is the myocardial layer area corresponding to the target cardiac chamber, $S_2$ is the area of the target cardiac chamber, and $L$ is the length of the target cardiac chamber.

According to a second aspect, an embodiment of the present invention further provides a device for determining a cardiac cycle, comprising:

an acquisition module, used for acquiring a cardiac ultrasound video;

a classification module, used for classifying the cardiac ultrasound video by using a section type recognition model to determine a section type of the cardiac ultrasound video; and

a cardiac cycle determination module, used for processing the cardiac ultrasound video by using a systole and diastole recognition model corresponding to the section type to obtain the cardiac cycle corresponding to the cardiac ultrasound video.

According to a third aspect, an embodiment of the present invention further provides ultrasonic equipment, comprising:

a memory and a processor which are in communication connection with each other, wherein computer instructions are stored in the memory, and the processor executes the method for determining the cardiac cycle according to the first aspect of the present invention or any implementation mode of the first aspect by executing the computer instructions.

According to a fourth aspect, an embodiment of the present invention further provides a computer-readable storage medium, the computer-readable storage medium stores computer instructions, and the computer instructions are used for causing a computer to execute the method for determining the cardiac cycle according to the first aspect of the present invention or any implementation mode of the first aspect.

**Brief Description of the Drawings**

In order to more clearly explain the specific implementation modes of the present invention or the technical solution in the prior art, the drawings needed in the description of the specific implementation modes or the prior art will be briefly introduced below. Obviously, the drawings in the following description are only some implementation modes of the present invention. For those of ordinary skill in the art, other drawings can be obtained according to the provided drawings without paying creative labor.

Fig. 1 is a flowchart of a method for determining a cardiac cycle according to an embodiment of the present invention;

Fig. 2 is a flowchart of a method for determining a cardiac cycle according to an embodiment of the present invention;

Fig. 3 is a structural diagram of a systole and diastole recognition model according to an embodiment of the present invention;

Fig. 4 is a structural diagram of a systole and diastole recognition model according to an embodiment of the present invention;

Fig. 5 is a flowchart of a method for training a systole and diastole recognition model according to an embodiment of the present invention;

Fig. 6 is a flowchart of a method for determining cardiac parameters according to an embodiment of the present invention;

Fig. 7 is a flowchart of a method for determining cardiac parameters according to an embodiment of the present invention;

Fig. 8 is a flowchart of a method for determining cardiac parameters according to an embodiment of the present invention;

Fig. 9 is a structural block diagram of a device for determining a cardiac cycle according to an embodiment of the present invention; and

Fig. 10 is a diagram of a hardware structure of ultrasonic equipment provided by an embodiment of the present invention.

**Detailed Description of the Embodiments**

In order to make the objective, technical solution and advantages of the embodiments of the present invention clearer, the technical solution in the embodiments of the present invention will be described clearly and completely below with reference to the drawings in the embodiments of the present invention. Obviously, the described embodiments are only part of the embodiments of the present invention, not all of the embodiments. Based on the embodiments of the present invention, all other embodiments obtained by those skilled in the art without creative labor are within the scope of the present invention.

It should be noted that a method for determining a cardiac cycle described in the embodiments of the present invention can be applied in the process of acquiring a cardiac ultrasound video. For example, ultrasonic equipment is used to acquire the cardiac ultrasound video, and the corresponding cardiac cycle is detected and corresponding cardiac parameters are determined. Or it can be applied in the subsequent analysis process of the cardiac ultrasound video, for example, the cardiac ultrasound video is stored in the ultrasonic equipment in advance, and when the cardiac ultrasound video needs to be analyzed, the method for determining the cardiac cycle described in the embodiment of the present invention is adopted to determine the corresponding cardiac cycle and the corresponding cardiac parameters.

According to an embodiment of the present invention, a method for determining a cardiac cycle is provided. It should be noted that the steps shown in the flowchart of the drawing can be executed in a computer system such as a set of computer-executable instructions, and although the logical sequence is shown in the flowchart, in some cases, the steps shown or described can be executed in a different sequence.

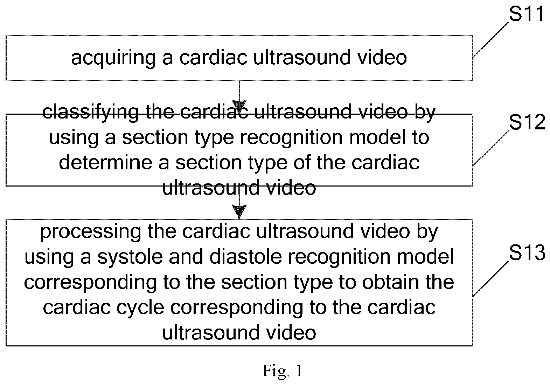

In this embodiment, a method for determining a cardiac cycle is provided, which can be used in ultrasonic equipment. Fig. 1 is a flowchart of a method for determining a cardiac cycle according to an embodiment of the present invention. As shown in Fig. 1, the flow comprises the following steps:

S11, a cardiac ultrasound video is acquired.

As mentioned above, the detection of the cardiac cycle can be performed when the ultrasonic equipment acquires the cardiac ultrasound video, or when the cardiac ultrasound video needs to be analyzed later. Therefore, the cardiac ultrasound video can by acquired by the ultrasonic equipment from a body surface of an objective to be detected in real time, imported from the outside, or stored in the ultrasonic equipment in advance, and so on. There is no restriction on the way to acquire the cardiac ultrasound video here, as long as it is ensured that the cardiac ultrasound video can be obtained when cardiac cycle detection is needed.

S12, the cardiac ultrasound video is classified by using a section type recognition model to determine a section type of the cardiac ultrasound video.

The cardiac ultrasound video is obtained by detecting the body surface of the objective to be detected through a probe. When detection is performed on the body surface of the objective to be detected, detection needs to be performed from multiple positions due to the position of the heart, so cardiac ultrasound videos corresponding to multiple sections can be obtained, and the section types can reflect the conditions of different positions of the heart.

Therefore, before detecting the cardiac cycle, the ultrasonic equipment needs to determine the section type of the cardiac ultrasound video. The section type recognition model is used to determine the section type of the cardiac ultrasound video.

The section type recognition model can be trained in advance or trained in real time when cardiac cycle detection is needed. The section type recognition model is used to classify the section type of the cardiac ultrasound video, so it can also be understood as a classification model. The input of the section type recognition model is the cardiac ultrasound video, and the output is the section type corresponding to the cardiac ultrasound video. There is no restriction on the specific structure and training process of the section type recognition model here, as long as it is ensured that the section type recognition model can classify the section type of the input cardiac ultrasound video.

For example, the section type may include parasternal long axis (PLAX), parasternal short axis (PSAX), apical 2 chamber (A2C), apical 3 chamber (A3C), apical 4 chamber (A4C), etc. The section type recognition model can be constructed based on a neural network model, and the output of the model can be a recognized category, and can also be multiple recognized categories and the probability corresponding to each category, based on which the section type of the input cardiac ultrasound video can be determined.

The specific structure and training process of the section type recognition model will be described in detail below.

S13, the cardiac ultrasound video is processed by using a systole and diastole recognition model corresponding to the section type to obtain the cardiac cycle corresponding to the cardiac ultrasound video.

Because different section types reflect the conditions of different positions of the heart and the systole and diastole are objective factors reflecting the heart conditions, different section types correspond to different systoles and diastoles; that is, the systole and diastole correspond to the section type. Therefore, the ultrasonic equipment needs to use the systole and diastole recognition model corresponding to the section type to process the cardiac ultrasound video.

As mentioned above, the cardiac cycle can be represented by adjacent end systoles and/or end diastoles, so the end systole and/or the end diastole in the cardiac ultrasound video can be recognized by using the systole and diastole recognition model.

In the method for determining the cardiac cycle provided in this embodiment, the model is used to process the cardiac ultrasound video to detect the corresponding cardiac cycle. Model detection can avoid the use of an electrocardiograph and simplify the detection of the cardiac cycle. Furthermore, real-time detection of the cardiac cycle can be realized during echocardiography.

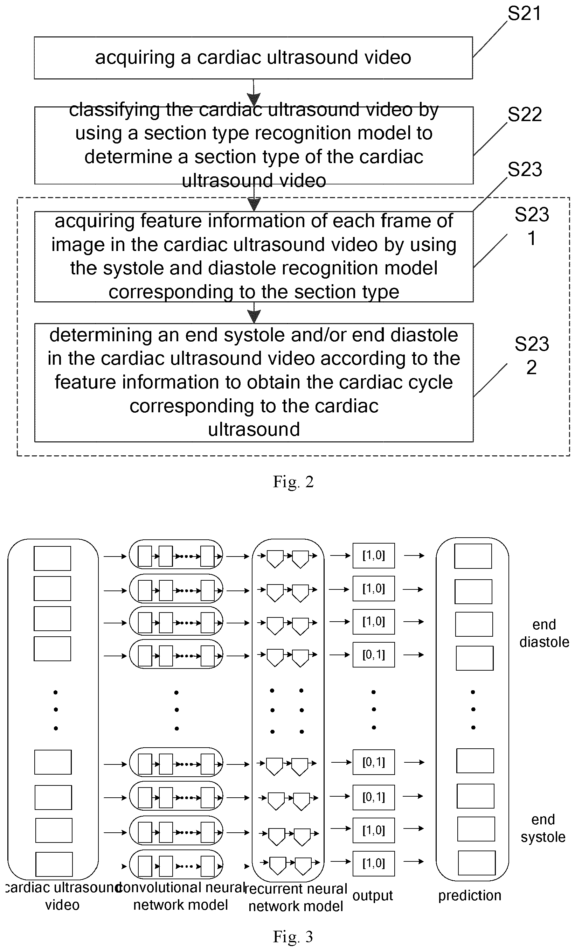

In this embodiment, a method for determining a cardiac cycle is further provided, which can be used in ultrasonic equipment. Fig. 2 is a flowchart of a method for determining a cardiac cycle according to an embodiment of the present invention. As shown in Fig. 2, the flow comprises the following steps:

S21, a cardiac ultrasound video is acquired.

Please refer to S11 of the embodiment shown in Fig. 1 for detail, which will not be repeated here.

S22, the cardiac ultrasound video is classified by using a section type recognition model to determine a section type of the cardiac ultrasound video.

The training process of the section type recognition model may comprise the following steps:

(1) ultrasonic equipment is used to acquire a cardiac ultrasound video of a standard section, wherein the type of the standard section may include parasternal long axis (PLAX), parasternal short axis (PSAX), apical 2 chamber (A2C), apical 3 chamber (A3C), apical 4 chamber (A4C), etc. The cardiac ultrasound video of the standard section is converted into continuous frames of images, which are stored in different folders according to the section type and an acquisition object.

For example, the ultrasonic equipment acquires video data of five standard sections (PLAX, PSAX, A2C, A3C, A4C), and five parent folders are set to represent the five sections. There are n subfolders in each of the five parent folders, wherein n is the number of acquisition objects, and the cardiac ultrasound videos of the n objects in each of the five sections are converted into continuous frames of images, which are saved in the corresponding folders.

(2) the continuous frames of images are read, and different labels are assigned to images of different section types.

According to actually collected data, enhancement processing with practical meaning is conducted to increase a sample size. For example, if some cardiac data images have larger contrast and some have smaller contrast, then appropriate contrast adjustment can be conducted on an original data set to generate more "enhanced" data sets.

Sample data are randomly scrambled, and a training set, a verification set and a test set are distributed in proper proportion.

(3) a classification neural network model is established, training is conducted on the training set for each iteration, and verification is conducted on the verification set.

Training is conducted till loss no longer decreases and the accuracy of the training set and the verification set reaches a high level, and finally, an accuracy rate is obtained on the test set and coefficients are saved. The assigned training set may not necessarily represent the whole data set, that is, some features of part of the whole data set are not trained on the training set, which leads to low predicted accuracy, so cross-validation is needed to reduce the representative problems of the training set as much as possible. At the same time, cross-validation is adopted to test the stability of a neural network for different training sets.

Images are re-read, the images with different sections are processed again in the same way as above (1) and (2) and corresponding labels are assigned thereto, then the order is disturbed and the above operations are repeated so as to obtain multiple accuracy rates and coefficients of the test set, and a coefficient with the highest accuracy rate is obtained through cross-validation.

The step that different labels are assigned to image data of different section types specifically comprises: since the training set is part of an image set and does not include all the images, it is hoped that cross-validation will increase the representativeness of the image set assigned to the training set after random scrambling. Under normal circumstances, a convolutional neural network needs to be trained with a set of data, which is input images plus corresponding labels. Classification means putting images with different sections into corresponding folders. After reading the images, for example, all images of A2C are labeled with (1, 0, 0, 0 ,0), images of A3C are labeled with (0, 1, 0, 0, 0), and so on.

Please refer to S12 of the embodiment shown in Fig. 1 for other details, which will not be repeated here.

S23, the cardiac ultrasound video is processed by using a systole and diastole recognition model corresponding to the section type to obtain the cardiac cycle corresponding to the cardiac ultrasound video.

Since the systole and diastole recognition model corresponds to the section type, after determining the section type, the ultrasonic equipment can extract the corresponding systole and diastole recognition model according to the section type, so as to detect the feature information of each frame of image in the cardiac ultrasound video. Specifically, the above S23 comprises the following steps:

S231, feature information of each frame of image in the cardiac ultrasound video is acquired by using the systole and diastole recognition model corresponding to the section type.

Specifically, the systole and diastole recognition model can be a classification model, the input of which is the cardiac ultrasound video, and the output is the feature information of each frame of image in the cardiac ultrasound video; that is, each frame of image in the cardiac ultrasound video is classified to determine whether it is a systolic image frame or a diastolic image frame. Or the systole and diastole recognition model can be a regression model simulating a relative volume coefficient of the cardiac chamber, the input of which is the cardiac ultrasound video, and the output is a coefficient of each frame of image in the cardiac ultrasound video, and the coefficient is used to indirectly indicate whether it is a systolic image frame or a diastolic image frame.

The ultrasonic equipment can use different labels to distinguish the section types, and the same labels as the corresponding section types are set in the systole and diastole recognition model. Then, the ultrasonic equipment can determine the systole and diastole recognition model corresponding to the section type by means of the label.

The ultrasonic equipment inputs the cardiac ultrasound video into the determined systole and diastole recognition model. Since the cardiac ultrasound video is composed of several continuous image frames, after inputting the cardiac ultrasound video into the systole and diastole recognition model, the model identifies the category of each image frame in the cardiac ultrasound video in sequence, thus determining the feature information of each frame of image.

The specific structure and training process of the systole and diastole recognition model are not limited here, as long as it is ensured that the feature information of each frame of image in the cardiac ultrasound video can be detected. The specific structure and training process will be described in detail below.

S232, an end systole and/or an end diastole are/is determined in the cardiac ultrasound video according to the feature information to obtain the cardiac cycle corresponding to the cardiac ultrasound video.

Because the cardiac cycle can be expressed by the adjacent end systoles or the adjacent end diastoles, the ultrasonic equipment can determine the end systole and the end diastole according to the feature information of each frame of image in the cardiac ultrasound video, and then detect the cardiac cycle corresponding to the cardiac ultrasound video.

The determination of end systole can be achieved by comparing the categories of two adjacent frames of images in sequence. When the categories of two adjacent frames of images change, it means that the previous frame of image is in the end systole or end diastole. For example, two adjacent frames of images are image frame A and image frame B, with the category of image frame A being systole and the category of image frame B being diastole. When the categories of image frame A and image frame B are compared, their categories change, then it can be determined that image frame A is an image frame of end systole.

When the ultrasonic equipment determines the end systole and the end diastole in the cardiac ultrasound video, the corresponding image frames can be labeled to form the cardiac cycle; or the cardiac cycle can be formed in other ways.

The feature information of each frame of image can be expressed in two forms, one is a preset identifier, and the other is a coefficient. These two forms and how to determine the end systole and/or the end diastole in the cardiac ultrasound video based on these two forms to obtain the cardiac cycle corresponding to the cardiac ultrasound video will be described in detail below.

1. Feature information being represented by preset identifier

In this embodiment, the feature information of each frame of image in the cardiac ultrasound video is represented by a preset identifier. A first preset identifier corresponds to the systole and a second preset identifier corresponds to the diastole.

Optionally, as shown in Fig. 3, the systole and diastole recognition model comprises an input end, a convolutional neural network model, a recurrent neural network model and an output end which are connected in sequence. It should be noted that although the convolutional neural network model and the recurrent neural network model shown in Fig. 3 are in one-to-one correspondence to the image frames of the input cardiac ultrasound video, it is only for explaining the processing of the model, but not for representing the actual architecture of the model. The systole and diastole model comprises the convolution neural network model and the recurrent neural network model, and the specific structural details of this model are not limited.

In Fig. 3, the first preset identifier is represented by [0,1], which is used to represent systole; the second preset identifier is represented by [1,0], which is used to represent diastole. However, other identifiers can be used according to actual needs.

The training process of the systole and diastole model shown in Fig. 3 may comprise the following steps:

(1) for continuous images of n objects in each of five sections, an appropriate sliding window and step size are selected for batch allocation of the continuous images according to GPU performance. For example, the first image to the thirtieth image form the first batch, the second image to the thirty-first image form the second batch, and so on, thus multiple batches are formed. According to the ECG tracing and the systolic and diastolic conditions of the left ventricle during image acquisition, each image is labeled as systole or diastole.

When the whole data set is divided into three categories: end diastole, end systole and others, the proportion of images of end diastole and end systole in the whole data set is very low. For example, if a cardiac cycle is 32 frames, the ratio of ES and ED is 1/16, which will lead to data imbalance and make model prediction more inclined to the category with higher proportion, resulting in misjudgment. Therefore, the problem of detecting ES and ED is changed into the problem of classifying the data set into two categories, which are systole and diastole, and the approximate duration ratio is 1: 2, thus relieving the problem of data imbalance. The last frame of image of systole (diastole) is the end systole (end diastole).

(2) a whole end-to-end model of 2D or 3D convolutional neural network CNN (feature extraction of left ventricle size in images) + bidirectional recurrent neural network RNN (temporal relationship extraction of consecutive frame features) + final fully connected layer FC is trained.

The model can also be understood as a classification model, which can be divided into two categories: systole and diastole. The difference from a section classification model is that the section classification model only uses 2D convolution neural network.

Accordingly, the above S232 may specifically comprise the following steps:

(1) the preset identifier corresponding to each frame of image is traversed, an image frame corresponding to the first preset identifier when the preset identifier experiences a change from the first preset identifier to the second preset identifier is determined as a first image frame, and/or an image frame corresponding to the second preset identifier when the preset identifier experiences a change from the second preset identifier to the first preset identifier is determined as a second image frame,

wherein the first image frame corresponds to the end systole and the second image frame corresponds to the end diastole.

The ultrasonic equipment traverses the preset identifier corresponding to each frame of image of the cardiac ultrasound video, and determines the corresponding image frame when the preset identifier changes. The cardiac ultrasound video may include a plurality of first image frames and a plurality of second image frames, and the cardiac cycle can be determined after the first image frames and/or the second image frames are determined.

(2) the cardiac cycle corresponding to the cardiac ultrasound video is detected based on the first image frame and/or the second image frame.

For example, with reference to Fig. 3, the output of each frame of image through the systole and diastole detection model is [0, 1] or [1, 0], which indicates that the frame of image is in systole or diastole. As shown in the upper half of Fig. 3, after continuous output of [1, 0], the output of the next frame of image becomes [0, 1], indicating that the image changes from diastole to systole, and then the last frame of image that outputs [1, 0] is in end diastole. When the model is tested, the last one predicted to be diastolic is ES, the last one predicted to be systolic is ED, and the time between two adjacent EDs or two adjacent ESs is the time of a single cardiac cycle.

2. Feature information being represented by coefficient

In this embodiment, the feature information of each frame of image in the cardiac ultrasound video is represented by a coefficient, which is used for representing the size of a target cardiac chamber in systole and diastole, and the coefficient increases progressively in diastole of the cardiac cycle and the coefficient decreases progressively in systole of the cardiac cycle. Particularly, the cardiac chamber includes left ventricle (LV), left atrium (LA), right ventricle (RV) and right atrium (RA), and the target cardiac chamber is one of them; for example, the target cardiac chamber can be left ventricle or left atrium, etc., which can be determined according to the actual situation.

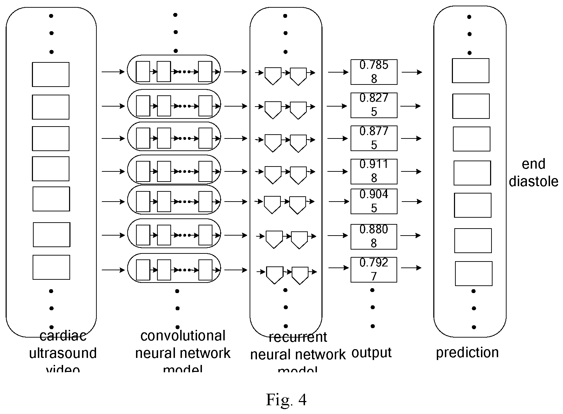

Optionally, as shown in Fig. 4, the systole and diastole recognition model comprises an input end, a convolutional neural network model, a recurrent neural network model and an output end which are connected in sequence. It should be noted that although the convolutional neural network model and the recurrent neural network model shown in Fig. 4 are in one-to-one correspondence to the image frames of the input cardiac ultrasound video, it is only for explaining the processing of the model, but not for representing the actual architecture of the model. The systole and diastole model comprises the convolution neural network model and the recurrent neural network model, and the specific structural details of this model are not limited.

The output of the systole and diastole recognition model is a coefficient, as shown in Fig. 4, the output of each frame of image is a coefficient, and the change trend of the coefficient is fixed. That is, when changing from systole to diastole, the coefficient gradually decreases, and when changing from diastole to systole, the coefficient gradually increases.

Accordingly, the above S232 comprises the following steps:

(1) the change trend of the coefficient is detected to determine a third image frame corresponding to the end systole and/or a fourth image frame corresponding to the end diastole.

Referring to Fig. 4, each frame of image of the cardiac ultrasound video corresponds to the output of a coefficient, and the change trend of the output coefficient corresponds to systole and diastole.

For example, in the whole process of systole, the output coefficient becomes smaller progressively; and in the whole process of diastole, the output coefficient becomes larger progressively.

(2) the cardiac cycle corresponding to the cardiac ultrasound video is detected based on the third image frame and/or the fourth image frame.

After detecting the end diastole and the end systole, the ultrasonic equipment can detect the cardiac cycle corresponding to the cardiac ultrasound video based on the corresponding image frame.

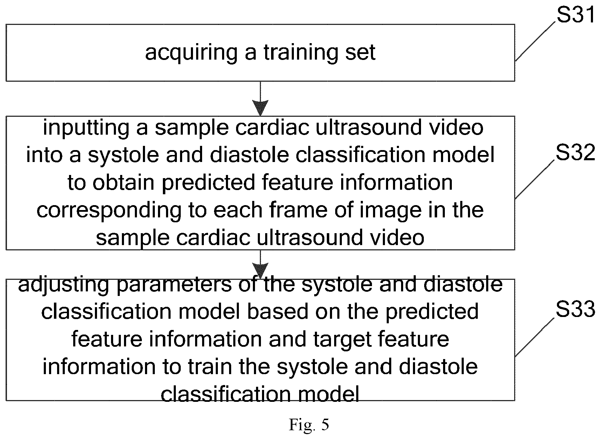

As an optional implementation mode of this embodiment, as shown in Fig. 5, the training process of the systole and diastole recognition model comprises the following steps:

S31, a training set is acquired.

The training set comprises a sample cardiac ultrasound video and labeled data, the labeled data includes target feature information corresponding to each frame of image in the sample cardiac ultrasound video, the feature information is represented by a sample coefficient, and the sample coefficient is used for representing the size of the target cardiac chamber in systole and diastole.

The sample coefficient can be determined artificially according to experience, or can be calculated by using an electrocardiographic tracing.

For example, the above S31 may comprise the following steps:

(1) a sample cardiac ultrasound video and a corresponding electrocardiographic tracing are acquired; and
(2) a sample coefficient corresponding to each frame of image in the sample cardiac ultrasound video is calculated at least based on the electrocardiographic tracing.

For example, the sample coefficient is calculated by the following formula:

$$y_t = \begin{cases} (\dfrac{\left| t - T_{ES}^i \right|}{\left| T_{ES}^i - T_{ED}^i \right|})^3, & if\ T_{ED}^i < t \le T_{ES}^i \\ (\dfrac{\left| t - T_{ES}^i \right|}{\left| T_{ES}^i - T_{ED}^{i+1} \right|})^{1/3}, & if\ T_{ES}^i < t \le T_{ED}^i \end{cases};$$

wherein i is the i-th cardiac cycle in the sample cardiac ultrasound video; t is the t-th frame of image in the sample cardiac ultrasound video; $T_{ED}^i$ is the time of end diastole in the i-th cardiac cycle; $T_{ES}^i$ is the time of end systole in the i-th cardiac cycle; and $y_t$ is the sample coefficient corresponding to the t-th frame of image in the sample cardiac ultrasound video.

S32, the sample cardiac ultrasound video is input into the systole and diastole recognition model to obtain predicted feature information corresponding to each frame of image in the sample cardiac ultrasound video.

By setting initial parameters for the systole and diastole recognition model and inputting the training sets into the systole and diastole recognition model in sequence, the predicted feature information corresponding to each frame of image in the sample cardiac ultrasound video can be obtained. The predicted feature information is represented by a prediction coefficient.

S33, parameters of the systole and diastole recognition model are adjusted based on the predicted feature information and the target feature information to train the systole and diastole recognition model.

The specific value of the predicted feature information obtained in S32 is related to parameters in the systole and diastole recognition model. Therefore, the parameters of the systole and diastole recognition model can be adjusted through the numerical relationship between the prediction coefficient and the sample coefficient, so as to realize the training of the systole and diastole recognition model.

As a specific implementation mode of the training process of the systole and diastole recognition model, the training process can be realized in the following way:

(1) for continuous images of n objects in each of five sections, an appropriate sliding window and step size are selected for batch allocation of the continuous images according to GPU performance (for example, the first image to the thirtieth image form the first batch, the second image to the thirty-first image form the second batch, and so on), thus multiple batches are formed.
According to the ECG tracing and the systolic and diastolic conditions of the left ventricle during image acquisition, each image is labeled as systole or diastole, and then the sample coefficient is determined according to the calculation formula of sample coefficient in S31, so as to assign a different value to each image, and the value simulates the size change of left ventricle in the cardiac cycle.
(2) a whole end-to-end model of 2D convolutional neural network CNN (feature extraction of left ventricle size in images) + bidirectional recurrent neural network RNN (temporal relationship extraction of consecutive frame features) + final regression layer (extraction of values simulating left ventricle size) is trained. When the model is tested, a series of continuous values (i.e., coefficients) are obtained from input sequential image sets. The values basically increase monotonically and then decrease monotonically in a repeated manner. The time point where the maximum value is located is the end diastole, and the time point where the minimum value is located is the end systole.

Optionally, the above S32 may comprise the following steps:

(1) a segmentation model corresponding to the section type is used to obtain the cardiac chamber in each frame of image through segmentation.

As shown above, different section types reflect different conditions of the heart, so it is necessary to adopt the segmentation model corresponding to the section type to obtain the cardiac chamber in each frame of image through segmentation. Cardiac chamber segmentation can be about segmentation of only the target cardiac chamber (for example, the left ventricle), or about segmentation of all the cardiac chambers, which will be determined according to the actual situation.

(2) the cardiac chamber obtained by segmentation and the cardiac ultrasound video are input into the determined systole and diastole recognition model to obtain the feature information of each frame of image in the cardiac ultrasound video.

By taking the cardiac chamber obtained by segmentation as one of the inputs of the model, other parts of the image unrelated to the detection of end systole and end diastole can be removed, and the classification accuracy is improved.

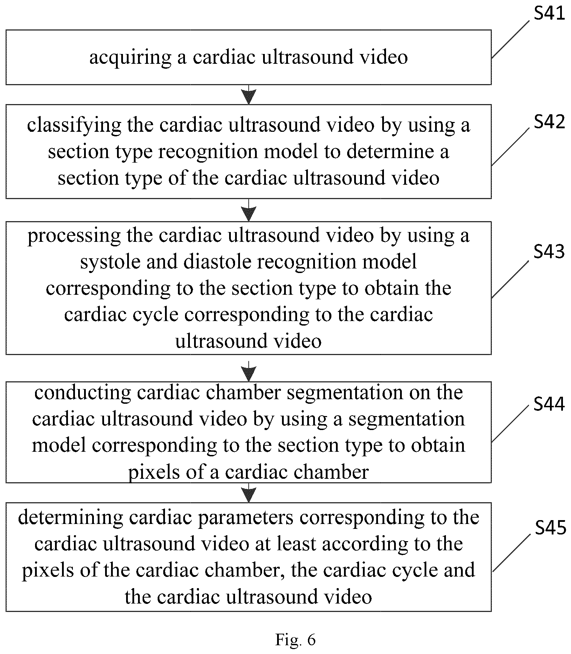

In this embodiment, a method for determining cardiac parameters is provided, which can be used in ultrasonic equipment. Fig. 6 is a flowchart of a method for determining cardiac parameters according to an embodiment of the present invention. As shown in Fig. 6, the flow comprises the following steps:

S41, a cardiac ultrasound video is acquired.

Please refer to S11 of the embodiment shown in Fig. 1 for the way to acquire the cardiac ultrasound video, which will not be repeated here.

S42, the cardiac ultrasound video is classified by using a section type recognition model to determine a section type of the cardiac ultrasound video.

Please refer to S12 or S22 of the embodiment shown in Fig. 1 or Fig. 2 for detail, which will not be repeated here.

S43, the cardiac ultrasound video is processed by using a systole and diastole recognition model corresponding to the section type to obtain the cardiac cycle corresponding to the cardiac ultrasound video.

Please refer to S13 or S23 of the embodiment shown in Fig. 1 or Fig. 2 for detail, which will not be repeated here.

S44, cardiac chamber segmentation is conducted on the cardiac ultrasound video by using a segmentation model corresponding to the section type to obtain pixels of a cardiac chamber.

As shown above, different section types reflect different conditions of the heart, so it is necessary to adopt the segmentation model corresponding to the section type to obtain the cardiac chamber in each frame of image through segmentation. Cardiac chamber segmentation can be about segmentation of only the target cardiac chamber (for example, the left ventricle), or about segmentation of all the cardiac chambers, which will be determined according to the actual situation. The ultrasonic equipment can obtain the pixels corresponding to each cardiac chamber after cardiac chamber segmentation.

S45, the cardiac parameters corresponding to the cardiac ultrasound video are determined at least according to the pixels of the cardiac chamber, the cardiac cycle and the cardiac ultrasound video.

The cardiac parameters include at least one of ejection fraction, end-diastolic volume, end-systolic volume and target cardiac chamber weight.

When the cardiac parameters are determined, for example, the ejection fraction can be calculated by the number of the pixels, the end-diastolic volume can be calculated by the area of the pixels and the cardiac cycle, and the target cardiac chamber weight can be calculated by the area of the pixels and the cardiac ultrasound video, and so on. The specific calculation method will be described below.

According to the method for determining the cardiac parameters provided by the embodiment, the pixels of each cardiac chamber can be obtained by classification of the cardiac ultrasound video and segmentation of the cardiac chamber, and then the cardiac parameters corresponding to the cardiac ultrasound video are determined based on the pixels of each cardiac chamber; that is to say, by processing the cardiac ultrasound video with the model, the accuracy of segmentation of each cardiac chamber in each frame of image of the cardiac ultrasound video is improved, and thus the accuracy of calculation of the cardiac parameters can be improved.

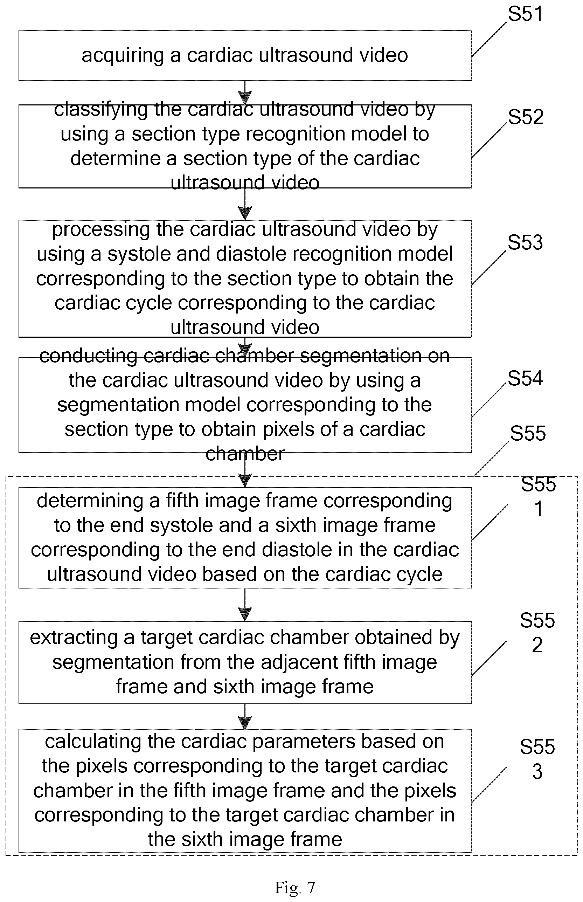

In this embodiment, a method for determining cardiac parameters is further provided, which can be used in ultrasonic equipment. Fig. 7 is a flowchart of a method for determining cardiac parameters according to an embodiment of the present invention. As shown in Fig. 7, the flow comprises the following steps:

S51, a cardiac ultrasound video is acquired.

Please refer to S41 of the embodiment shown in Fig. 6 for detail, which will not be repeated here.

S52, the cardiac ultrasound video is classified by using a section type recognition model to determine a section type of the cardiac ultrasound video.

Please refer to S42 of the embodiment shown in Fig. 6 for detail, which will not be repeated here.

S53, the cardiac ultrasound video is processed by using a systole and diastole recognition model corresponding to the section type to obtain the cardiac cycle corresponding to the cardiac ultrasound video.

Please refer to S43 of the embodiment shown in Fig. 6 for detail, which will not be repeated here.

S54, cardiac chamber segmentation is conducted on the cardiac ultrasound video by using a segmentation model corresponding to the section type to obtain pixels of a cardiac chamber.

Please refer to S44 of the embodiment shown in Fig. 6 for detail, which will not be repeated here.

S55, the cardiac parameters corresponding to the cardiac ultrasound video are determined at least according to the pixels of the cardiac chamber, the cardiac cycle and the cardiac ultrasound video.

The cardiac parameters include at least one of ejection fraction, end-diastolic volume, end-systolic volume and target cardiac chamber weight.

In this embodiment, the cardiac parameters to be determined are ejection fraction, end-diastolic volume and end-systolic volume. Specifically, S55 may comprise the following steps:

S551, a fifth image frame corresponding to the end systole and a sixth image frame corresponding to the end diastole in the cardiac ultrasound video are determined based on the cardiac cycle.

Because the cardiac cycle is determined by the adjacent end diastoles or the adjacent end systoles, the fifth image frame corresponding to the end systole and the sixth image frame corresponding to the end diastole in the cardiac ultrasound video can be easily determined through the cardiac cycle. Furthermore, the cardiac ultrasound video may include a plurality of cardiac cycles, that is, a cardiac ultrasound video includes a plurality of fifth image frames and sixth image frames.

S552, a target cardiac chamber obtained by segmentation is extracted from the adjacent fifth image frame and sixth image frame.

The ultrasonic equipment has conducted cardiac chamber segmentation in S54, and after determining the corresponding image frames in S551, it only needs to extract the target cardiac chambers, obtained by segmentation, corresponding to the image frames.

S553, the cardiac parameters are calculated based on the pixels corresponding to the target cardiac chamber in the fifth image frame and the pixels corresponding to the target cardiac chamber in the sixth image frame.

Specifically, (1) the ejection fraction is calculated as follows:

(1.1) the number of the pixels corresponding to the target cardiac chamber in the fifth image frame and the number of the pixels corresponding to the target cardiac chamber in the sixth image frame are counted; and
(1.2) the ejection fraction is calculated by using the counted numbers of the pixels.

For example, it can be calculated by the following formula:

$$\text{ejection fraction} = \frac{Pix_{ED} - Pix_{ES}}{Pix_{ED}} * 100\% \ ;$$

wherein $Pix_{ED}$ is the number of the pixels corresponding to the target cardiac chamber in the sixth image frame, and $Pix_{ES}$ is the number of the pixels corresponding to the target cardiac chamber in the fifth image frame.

(2) The end-diastolic volume and end-systolic volume are calculated as follows:

(2.1) the number of the pixels corresponding to the target cardiac chamber in the fifth image frame and the number of the pixels corresponding to the target cardiac chamber in the sixth image frame are counted.

(2.2) an end-diastolic area of the target cardiac chamber and an end-systolic area of the target cardiac chamber are determined by using the counted numbers of the pixels.

The counted numbers of the pixels correspond to the number of the pixels in the target cardiac chamber in end diastole and the number of the pixels in the target cardiac chamber in end systole, respectively. As for the area, the number of the pixels can be multiplied by the actual area occupied by each pixel, wherein the actual area occupied by each pixel is determined according to a machine acquiring the cardiac ultrasound video, and the value is fixed. Specifically, the ultrasonic equipment multiplies the number of the pixels of the target cardiac chamber in end diastole by the actual area of each pixel to obtain the end-diastolic area of the target cardiac chamber, and multiplies the number of the pixels of the target cardiac chamber in end systole by the actual area of each pixel to obtain the end-systolic area of the target cardiac chamber.

(2.3) linear fitting is performed on the pixels corresponding to the target cardiac chamber in the fifth image frame and the pixels corresponding to the target cardiac chamber in the sixth image frame to determine a target cardiac chamber length corresponding to the end diastole and a target cardiac chamber length corresponding to the end systole.

Specifically, the target cardiac chamber obtained by segmentation can be regarded as an image, a coordinate axis is established with the lower left corner of the image obtained by segmentation as the origin, each pixel of the target cardiac chamber corresponds to a two-dimensional coordinate in the coordinate axis, and a linear function y=ax+b can be established to fit all pixels; and after obtaining the linear function, the line segment of the overlapping part between the linear function and the pixels is the line segment length, that is, the target cardiac chamber length. The target cardiac chamber length varies at end diastole and end systole, so it needs to be calculated separately.

(2.4) an end-diastolic volume of the target cardiac chamber is calculated based on the end-diastolic area of the target cardiac chamber and the target cardiac chamber length corresponding to the end diastole; or an end-systolic volume of the target cardiac chamber is calculated based on the end-systolic area of the target cardiac chamber and the target cardiac chamber length corresponding to the end systole.

Specifically, it can be calculated by the following formula:

$$\text{end-diastolic volume of target cardiac chamber} = \frac{0.85 * S_{ED}^{2}}{L_{ED}};$$

$$\text{end-systolic volume of target cardiac chamber} = \frac{0.85 * S_{ES}^{2}}{L_{ES}};$$

wherein $S_{ED}$ is the end-diastolic area of the target cardiac chamber, $L_{ED}$ is the target cardiac chamber length corresponding to the end diastole, $S_{ES}$ is the end-systolic area of the target cardiac chamber, and $L_{ES}$ is the target cardiac chamber length corresponding to the end systole.

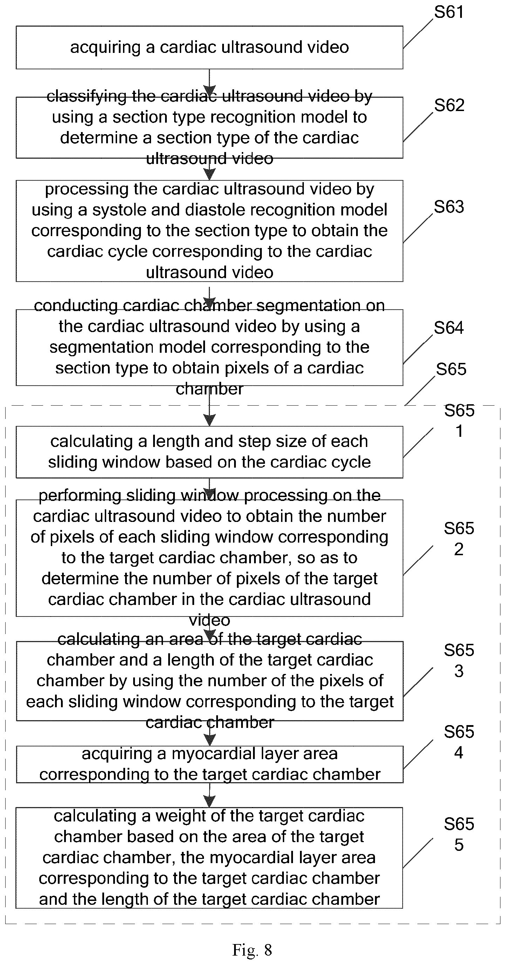

In this embodiment, a method for determining cardiac parameters is further provided, which can be used in ultrasonic equipment. Fig. 8 is a flowchart of a method for determining cardiac parameters according to an embodiment of the present invention. As shown in Fig. 8, the flow comprises the following steps:

S61, a cardiac ultrasound video is acquired.

Please refer to S51 of the embodiment shown in Fig. 7 for detail, which will not be repeated here.

S62, the cardiac ultrasound video is classified by using a section type recognition model to determine a section type of the cardiac ultrasound video.

Please refer to S52 of the embodiment shown in Fig. 7 for detail, which will not be repeated here.

S63, the cardiac ultrasound video is processed by using a systole and diastole recognition model corresponding to the section type to obtain the cardiac cycle corresponding to the cardiac ultrasound video.

Please refer to S53 of the embodiment shown in Fig. 7 for detail, which will not be repeated here.

S64, cardiac chamber segmentation is conducted on the cardiac ultrasound video by using a segmentation model corresponding to the section type to obtain pixels of a cardiac chamber.

Please refer to S54 of the embodiment shown in Fig. 7 for detail, which will not be repeated here.

S65, the cardiac parameters corresponding to the cardiac ultrasound video are determined at least according to the pixels of the cardiac chamber, the cardiac cycle and the cardiac ultrasound video.

The cardiac parameters include at least one of ejection fraction, end-diastolic volume, end-systolic volume and target cardiac chamber weight.

In this embodiment, the cardiac parameter to be determined is target cardiac chamber weight. Specifically, S65 comprises the following steps:

S651, a length and step size of sliding windows are calculated based on the cardiac cycle.

The cardiac cycle represents the time of the periodic action of the heart, and the length and step size of the sliding windows can be determined according to the actual situation. For example, the length of the sliding window may be 90% of the cardiac cycle and the step size may be 50% of the cardiac cycle.

S652, sliding window processing is performed on the cardiac ultrasound video to obtain the number of pixels of each sliding window corresponding to the target cardiac chamber, so as to determine the number of pixels of the target cardiac chamber in the cardiac ultrasound video.

The ultrasonic equipment performs sliding window processing on the cardiac ultrasound video. For example, it is determined that the section type of the cardiac ultrasound video is A4C, and the object of the sliding window here is continuous images contained in some A4C section video. For example, if the captured video lasts for 4 seconds and the frame rate is 32 frames/second, then the video includes 128 frames of images. Such processing is conducted on each A4C sample video, which is followed by sliding window processing. For example, if the cardiac cycle is 10 frames, the window length is 9 frames and the step size is 5 frames, then it is determined, after using the sliding window, that the first to ninth frames of the video form a first window, and the sixth to fourteenth frames form a second window.

Specifically, S652 may comprise the following steps:

(1) sliding is performed on the cardiac ultrasound video based on the step size to determine target cardiac chambers included in each sliding window, wherein each sliding window comprises at least one image frame of the cardiac ultrasound video.

As mentioned in the above example, the cardiac ultrasound video includes 128 frames of images, the window length is 9 frames, the step size is 5 frames, the first to ninth frames of the video form a first window, and the sixth to fourteenth frames form a second window. Because the first window includes the first frame to the ninth frame of the video, and each frame of image is considered to include the target cardiac chamber, the first window includes nine target cardiac chambers. By analogy, each window includes nine target cardiac chambers.

(2) the numbers of pixels of target cardiac chambers included in each sliding window are compared to obtain the number of pixels of each sliding window corresponding to the target cardiac chamber.

Since each sliding window includes nine target cardiac chambers, the sizes of the target cardiac chambers are different at different time points, that is, the numbers of pixels included in the target cardiac chambers are different at different time periods. In this embodiment, by comparing the numbers of pixels included in nine target cardiac chambers in each window, the unique number of pixels corresponding to target information for the window is determined.

For example, the numbers of pixels included in the nine target cardiac chambers included in each window can be ranked, and the maximum value or the value being above 90% of the numbers of pixels can be taken as the number of pixels corresponding to the target information for the window. It can be set according to the actual situation, as long as it is ensured that one number is determined from the numbers of pixels included in the nine target cardiac chambers as the number of pixels corresponding to the target cardiac chamber for the window.

(3) the median of the numbers of pixels of each sliding window corresponding to the target cardiac chamber is taken to obtain the number of the pixels of the target cardiac chamber in the cardiac ultrasound video.

After traversing the whole cardiac ultrasound video by the ultrasonic equipment by using sliding windows, each window corresponds to the number of pixels of one target cardiac chamber. After traversing, multiple numbers of pixels corresponding to the target cardiac chambers can be obtained, and the median can be taken to obtain the number of pixels of the target cardiac chamber in the cardiac ultrasound video.

S653, an area of the target cardiac chamber and a length of the target cardiac chamber are calculated by using the number of the pixels of each sliding window corresponding to the target cardiac chamber.

After determining the number of pixels of the target cardiac chamber in the cardiac ultrasound video in S652, the area of the target cardiac chamber can be obtained by multiplying the number of pixels by the actual area of each pixel.

The length of the target cardiac chamber can also be determined in the same way as determining the number of pixels of the target cardiac chamber. For example, the lengths of the target cardiac chambers corresponding to each window are first determined, and then the length of the target cardiac chamber in the cardiac ultrasound video is determined. For the lengths of the target cardiac chambers corresponding to each window, the length of the target cardiac chamber in each image frame included in each window can be calculated first (which can be determined by linear fitting as described above), then the length of the only target cardiac chamber corresponding to each window can be determined through ranking and comparison, and finally, the median of the lengths of the target cardiac chambers corresponding to all windows can be taken to obtain the length of the target cardiac chamber in the cardiac ultrasound video.

S654, a myocardial layer area corresponding to the target cardiac chamber is acquired.

The myocardial layer area corresponding to the target cardiac chamber is the product of the number of pixels of a myocardial layer corresponding to the target cardiac chamber in the cardiac ultrasound video and an area of each pixel.

The myocardial layer area corresponding to the target cardiac chamber can be obtained from the outside or determined by the same method as the area of the target cardiac chamber. For example, a segmentation model corresponding to the section type can be used to obtain the myocardial layer corresponding to the target cardiac chamber by segmentation, that is, myocardial layer segmentation corresponding to the target cardiac chamber is conducted on each frame of image in the cardiac ultrasound video, and then the number of pixels of the myocardial layer corresponding to the target cardiac chamber in the cardiac ultrasound video is determined by S551-S552, and the myocardial layer area corresponding to the target cardiac chamber can be obtained by multiplying the actual area of pixels by the corresponding number of pixels.

S655, a weight of the target cardiac chamber is calculated based on the area of the target cardiac chamber, the myocardial layer area corresponding to the target cardiac chamber and the length of the target cardiac chamber.

Specifically, the weight of the target cardiac chamber can be calculated by the following formula:

weight of target cardiac chamber=

$$1.05 * ( ( 5/6 ) * (S_1 + S_2) * (L + t) - ( ( 5/6 ) * S_2 * L) ;$$

$$t = \sqrt{(S_1 + S_2)/\pi} - \sqrt{S_2/\pi} ;$$

wherein $S_1$ is the myocardial layer area corresponding to the target cardiac chamber, $S_2$ is the area of the target cardiac chamber, and $L$ is the length of the target cardiac chamber.

In this embodiment, a device for determining a cardiac cycle and a method for determining cardiac parameters are also provided. The device is used to realize the above embodiments and preferred implementation modes, and repeated parts will not be described again. As used below, the term "module" may be a combination of software and/or hardware that implements a predetermined function. Although the device described in the following embodiments is preferably implemented in software, the implementation of hardware, or a combination of software and hardware, is also possible and contemplated.

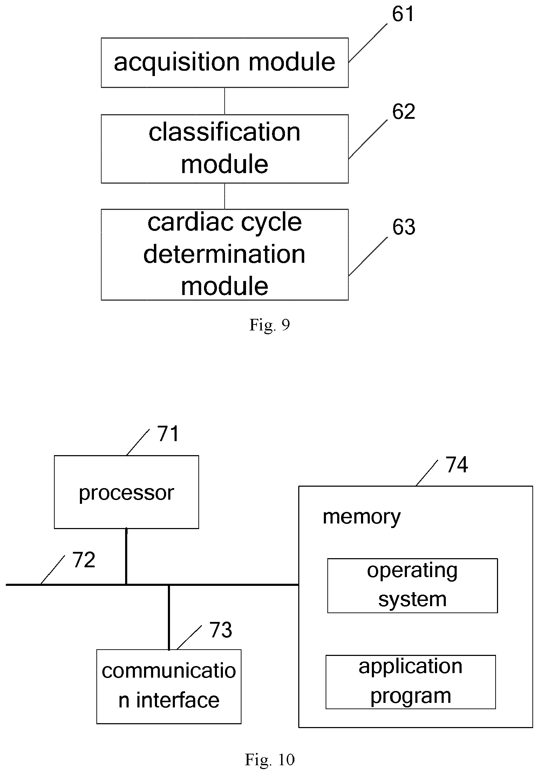

The embodiment provides a device for determining a cardiac cycle, as shown in Fig. 9, comprising:

an acquisition module 61, used for acquiring a cardiac ultrasound video;
a classification module 62, used for classifying the cardiac ultrasound video by using a section type recognition model to determine a section type of the cardiac ultrasound video; and
a cardiac cycle determination module 63, used for processing the cardiac ultrasound video by using a systole and diastole recognition model corresponding to the section type to obtain the cardiac cycle corresponding to the cardiac ultrasound video.

The device for determining the cardiac cycle or the device for determining the cardiac parameters in this embodiment is presented in the form of a functional unit, wherein the unit refers to ASIC, processor executing one or more software or fixed programs and memory, and/or other devices that can provide the above functions.

Further functional descriptions of the above-mentioned modules are the same as those of the above-mentioned corresponding embodiments, and will not be repeated here.

An embodiment of the present invention also provides ultrasonic equipment, which has the device for determining the cardiac cycle as shown in Fig. 9.

Please refer to Fig. 10, which is a structural diagram of ultrasonic equipment provided by an optional embodiment of the present invention. As shown in Fig. 10, the ultrasonic equipment may comprise at least one processor 71, such as a central processing unit (CPU), at least one communication interface 73, a memory 74, and at least one communication bus 72. The communication bus 72 is used to realize the communication between these components. The communication interface 73 may include a display and a keyboard, and optionally, the communication interface 73 may also include a standard wired interface and a wireless interface. The memory 74 may be a high-speed volatile random access memory (RAM) or a non-volatile memory, such as at least one disk memory. Optionally, the memory 74 may also be at least one storage device located far from the aforementioned processor 71. The processor 71 can be combined with the device described in Fig. 9, an application program is stored in the memory 74, and the processor 71 calls a program code stored in the memory 74 for executing any of the above method steps.

The communication bus 72 may be peripheral component interconnect (PCI) bus or extended industry standard architecture (EISA) bus, etc. The communication bus 72 can be divided into address bus, data bus, control bus, etc. For convenience of illustration, it is represented by one thick line only in Fig. 10, but it does not mean that there is only one bus or one type of buses.

The memory 74 may include a volatile memory, such as random-access memory (RAM). The memory may also include a non-volatile memory, such as flash memory, hard disk drive (HDD) or solid-state drive (SSD). The memory 74 may also include a combination of the above memories.

The processor 71 may be a central processing unit (CPU), a network processor (NP) or a combination of CPU and NP.

The processor 71 may further include a hardware chip. The hardware chip may be application-specific integrated circuit (ASIC), programmable logic device (PLD) or a combination thereof. The PLD may be complex programmable logic device (CPLD), field-programmable gate array (FPGA), generic array logic (GAL) or any combination thereof.

Optionally, the memory 74 is also used to store program instructions. The processor 71 can call the program instructions to realize the method for determining the cardiac cycle as shown in the embodiments of Figs. 1 to 8 of the present application.

An embodiment of the present invention also provides a non-transient computer storage medium, wherein the computer storage medium stores computer-executable instructions, and the computer-executable instructions can execute the method for determining the cardiac cycle or the method for determining the cardiac parameters in any of the above method embodiments. The storage medium may be magnetic disk, optical disk, read-only memory (ROM), random access memory (RAM), flash memory, hard disk drive (HDD) or solid-state drive (SSD), etc. The storage medium may also include a combination of the above memories.

Although the embodiments of the present invention have been described with reference to the accompanying drawings, various modifications and variations can be made by those skilled in the art without departing from the spirit and scope of the present invention, and such modifications and variations fall within the scope defined by the appended claims.

**Claims**

1. A method for determining a cardiac cycle, **characterized by** comprising:

   acquiring a cardiac ultrasound video;
   classifying the cardiac ultrasound video by using a section type recognition model to determine a section type of the cardiac ultrasound video; and
   processing the cardiac ultrasound video by using a systole and diastole recognition model corresponding to the section type to obtain the cardiac cycle corresponding to the cardiac ultrasound video.

2. The method according to claim 1, **characterized by** further comprising:

   conducting cardiac chamber segmentation on the cardiac ultrasound video by using a segmentation model corresponding to the section type to obtain pixels of a cardiac chamber; and
   determining cardiac parameters corresponding to the cardiac ultrasound video at least according to pixels of the cardiac chamber and the cardiac cycle, wherein the cardiac parameters include at least one of ejection fraction, end-diastolic volume, end-systolic volume and target cardiac chamber weight.

3. The method according to claim 1 or 2, wherein the step of processing the cardiac ultrasound video by using the systole and diastole recognition model corresponding to the section type to obtain the cardiac cycle corresponding to the cardiac ultrasound video comprises:

   acquiring feature information of each frame of image in the cardiac ultrasound video by using the systole and diastole recognition model corresponding to the section type; and
   determining an end systole and/or an end diastole in the cardiac ultrasound video according to the feature information to obtain the cardiac cycle corresponding to the cardiac ultrasound video.

4. The method according to claim 3, **characterized in that** the feature information of each frame of image in the cardiac ultrasound video is represented by a preset identifier, wherein a first preset identifier corresponds to systole and a second preset identifier corresponds to diastole; and
   the step of determining the end systole and/or the end diastole in the cardiac ultrasound video according to the feature information to obtain the cardiac cycle corresponding to the cardiac ultrasound video comprises:

   traversing the preset identifier corresponding to each frame of image, determining an image frame corresponding to the first preset identifier when the preset identifier experiences a change from the first preset identifier to the second preset identifier as a first image frame, and/or determining an image frame corresponding to the second preset identifier when the preset identifier experiences a change from the second preset identifier to the first preset identifier as a second image frame, wherein the first image frame corresponds to the end systole and the second image frame corresponds to the end diastole; and
   detecting the cardiac cycle corresponding to the cardiac ultrasound video based on the first image frame and/or the second image frame.

5. The method according to claim 3, **characterized in that** the feature information of each frame of image in the cardiac ultrasound video is represented by a coefficient, the coefficient is used to indicate a size of a target cardiac chamber in systole and diastole, the coefficient increases progressively in the diastole of the cardiac cycle and the coefficient decreases progressively in the systole of the cardiac cycle; and

the step of determining the end systole and/or the end diastole in the cardiac ultrasound video according to the feature information to obtain the cardiac cycle corresponding to the cardiac ultrasound video comprises:

detecting a size change of the coefficient to determine a third image frame corresponding to the end systole and/or a fourth image frame corresponding to the end diastole; and

detecting the cardiac cycle corresponding to the cardiac ultrasound video based on the third image frame and/or the fourth image frame.

6. The method according to claim 3, **characterized in that** the systole and diastole recognition model is trained in the following way:

acquiring a training set, wherein the training set comprises a sample cardiac ultrasound video and labeled data, the labeled data includes target feature information corresponding to each frame of image in the sample cardiac ultrasound video, the feature information is represented by a sample identifier or a sample coefficient, a first sample identifier corresponds to the systole and a second sample identifier corresponds to the diastole, and the sample coefficient is used for representing the size of the target cardiac chamber in the systole and the diastole;

inputting the sample cardiac ultrasound video into the systole and diastole recognition model to obtain predicted feature information corresponding to each frame of image in the sample cardiac ultrasound video; and

adjusting parameters of the systole and diastole recognition model based on the predicted feature information and the target feature information to train the systole and diastole recognition model.

7. The method according to claim 6, **characterized in that** when the labeled data are expressed by the sample coefficient, the sample coefficient is calculated by the following method:

acquiring an electrocardiographic tracing corresponding to the sample cardiac ultrasound video; and

calculating the sample coefficient corresponding to each frame of image in the sample cardiac ultrasound video at least based on the electrocardiographic tracing.

8. The method according to claim 2, **characterized in that** the step of determining the cardiac parameters corresponding to the cardiac ultrasound video at least according to the pixels of the cardiac chamber and the cardiac cycle comprises:

determining a fifth image frame corresponding to the end systole and a sixth image frame corresponding to the end diastole in the cardiac ultrasound video based on the cardiac cycle;

extracting a target cardiac chamber obtained by segmentation from the adjacent fifth image frame and sixth image frame; and

calculating the cardiac parameters based on pixels corresponding to the target cardiac chamber in the fifth image frame and pixels corresponding to the target cardiac chamber in the sixth image frame.

9. The method according to claim 8, **characterized in that** the step of calculating the cardiac parameters based on the pixels corresponding to the target cardiac chamber in the fifth image frame and the pixels corresponding to the target cardiac chamber in the sixth image frame comprises:

counting the number of the pixels corresponding to the target cardiac chamber in the fifth image frame and the number of the pixels corresponding to the target cardiac chamber in the sixth image frame;

calculating the ejection fraction by using the counted numbers of the pixels;

or,

determining an end-diastolic area of the target cardiac chamber and an end-systolic area of the target cardiac chamber by using the counted numbers of the pixels;

performing linear fitting on the pixels corresponding to the target cardiac chamber in the fifth image frame and the pixels corresponding to the target cardiac chamber in the sixth image frame to determine a target cardiac chamber length corresponding to the end diastole and a target cardiac chamber length corresponding to the end systole; and

calculating an end-diastolic volume of the target cardiac chamber based on the end-diastolic area of the target cardiac chamber and the target cardiac chamber length corresponding to the end diastole; or calculating an end-systolic volume of the target cardiac chamber based on the end-systolic area of the target cardiac chamber and the target cardiac chamber length corresponding to the end systole.

10. The method according to claim 2, **characterized in that** the step of determining the cardiac parameters corresponding to the cardiac ultrasound video at least according to the pixels of the cardiac chamber and the cardiac cycle comprises:

calculating a length and step size of sliding windows based on the cardiac cycle;
performing sliding window processing on the cardiac ultrasound video to obtain the number of pixels of each of the sliding windows corresponding to the target cardiac chamber, so as to determine the number of pixels of the target cardiac chamber in the cardiac ultrasound video;
calculating an area of the target cardiac chamber and a length of the target cardiac chamber by using the number of the pixels of each of the sliding windows corresponding to the target cardiac chamber;
acquiring a myocardial layer area corresponding to the target cardiac chamber, wherein the myocardial layer area corresponding to the target cardiac chamber is a product of the number of pixels of a myocardial layer corresponding to the target cardiac chamber in the cardiac ultrasound video and an area of each pixel; and
calculating a weight of the target cardiac chamber based on the area of the target cardiac chamber, the myocardial layer area corresponding to the target cardiac chamber and the length of the target cardiac chamber.

11. The method according to claim 10, **characterized in that** the step of performing sliding window processing on the cardiac ultrasound video to obtain the number of the pixels of each of the sliding windows corresponding to the target cardiac chamber, so as to determine the number of the pixels of the target cardiac chamber in the cardiac ultrasound video comprises:

sliding on the cardiac ultrasound video based on the step size to determine target cardiac chambers included in each of the sliding windows, wherein each of the sliding windows comprises at least one image frame of the cardiac ultrasound video;
comparing the numbers of pixels of the target cardiac chambers included in each of the sliding windows to obtain the number of pixels of each of the sliding windows corresponding to the target cardiac chamber; and
taking a median of the numbers of pixels of each of the sliding windows corresponding to the target cardiac chamber to obtain the number of the pixels of the target cardiac chamber in the cardiac ultrasound video.

12. Ultrasonic equipment, **characterized by** comprising:
a memory and a processor, the memory and the processor being in communication connection with each other, wherein computer instructions are stored in the memory, and the processor executes the method for determining the cardiac cycle according to any one of claims 1-11 by executing the computer instructions.

13. A computer-readable storage medium, **characterized in that** the computer-readable storage medium stores computer instructions, and the computer instructions are used for causing a computer to execute the method for determining the cardiac cycle according to any one of claims 1-11.

S11

acquiring a cardiac ultrasound video

classifying the cardiac ultrasound video by
using a section type recognition model to
determine a section type of the cardiac
ultrasound video

S12

processing the cardiac ultrasound video by
using a systole and diastole recognition model
corresponding to the section type to obtain the
cardiac cycle corresponding to the cardiac
ultrasound video

S13

Fig. 1

S21

acquiring a cardiac ultrasound video

S22

classifying the cardiac ultrasound video by using a section type recognition model to determine a section type of the cardiac ultrasound video

S23

acquiring feature information of each frame of image in the cardiac ultrasound video by using the systole and diastole recognition model corresponding to the section type

S23 1

determining an end systole and/or end diastole in the cardiac ultrasound video according to the feature information to obtain the cardiac cycle corresponding to the cardiac ultrasound

S23 2

Fig. 2

[1,0]
[1,0]
[1,0]
[0,1]

end diastole

[0,1]
[0,1]
[1,0]
[1,0]

end systole

cardiac ultrasound video    convolutional neural network model    recurrent neural network model    output    prediction

Fig. 3

Fig. 4

end
diastole

cardiac ultrasound video

convolutional neural network model

recurrent neural network model

output

prediction

0.7858
0.8275
0.8775
0.9118
0.9045
0.8808
0.7927

acquiring a training set

S31

inputting a sample cardiac ultrasound video into a systole and diastole classification model to obtain predicted feature information corresponding to each frame of image in the sample cardiac ultrasound video

S32

adjusting parameters of the systole and diastole classification model based on the predicted feature information and target feature information to train the systole and diastole classification model

S33

Fig. 5

S41

acquiring a cardiac ultrasound video

classifying the cardiac ultrasound video by using a
section type recognition model to determine a
section type of the cardiac ultrasound video

S42

processing the cardiac ultrasound video by using a
systole and diastole recognition model
corresponding to the section type to obtain the
cardiac cycle corresponding to the cardiac
ultrasound video

S43

conducting cardiac chamber segmentation on the
cardiac ultrasound video by using a segmentation
model corresponding to the section type to obtain
pixels of a cardiac chamber

S44

determining cardiac parameters corresponding to the
cardiac ultrasound video at least according to the
pixels of the cardiac chamber, the cardiac cycle and
the cardiac ultrasound video

S45

Fig. 6

acquiring a cardiac ultrasound video — S51

classifying the cardiac ultrasound video by using a section type recognition model to determine a section type of the cardiac ultrasound video — S52

processing the cardiac ultrasound video by using a systole and diastole recognition model corresponding to the section type to obtain the cardiac cycle corresponding to the cardiac ultrasound video — S53

conducting cardiac chamber segmentation on the cardiac ultrasound video by using a segmentation model corresponding to the section type to obtain pixels of a cardiac chamber — S54 S55

determining a fifth image frame corresponding to the end systole and a sixth image frame corresponding to the end diastole in the cardiac ultrasound video based on the cardiac cycle — S55 S55 1

extracting a target cardiac chamber obtained by segmentation from the adjacent fifth image frame and sixth image frame — S55 2

calculating the cardiac parameters based on the pixels corresponding to the target cardiac chamber in the fifth image frame and the pixels corresponding to the target cardiac chamber in the sixth image frame — S55 3

Fig. 7

acquiring a cardiac ultrasound video — S61

classifying the cardiac ultrasound video by using a section type recognition model to determine a section type of the cardiac ultrasound video — S62

processing the cardiac ultrasound video by using a systole and diastole recognition model corresponding to the section type to obtain the cardiac cycle corresponding to the cardiac ultrasound video — S63

conducting cardiac chamber segmentation on the cardiac ultrasound video by using a segmentation model corresponding to the section type to obtain pixels of a cardiac chamber — S64

S65

S65

calculating a length and step size of each sliding window based on the cardiac cycle — S65 1

performing sliding window processing on the cardiac ultrasound video to obtain the number of pixels of each sliding window corresponding to the target cardiac chamber, so as to determine the number of pixels of the target cardiac chamber in the cardiac ultrasound video — S65 2

calculating an area of the target cardiac chamber and a length of the target cardiac chamber by using the number of the pixels of each sliding window corresponding to the target cardiac chamber — S65 3

acquiring a myocardial layer area corresponding to the target cardiac chamber — S65 4

calculating a weight of the target cardiac chamber based on the area of the target cardiac chamber, the myocardial layer area corresponding to the target cardiac chamber and the length of the target cardiac chamber — S65 5

Fig. 8

acquisition module — 61

classification module — 62

cardiac cycle determination module — 63

Fig. 9

71 processor

72

73 communication interface

74 memory

operating system

application program

Fig. 10

## INTERNATIONAL SEARCH REPORT

| International application No. |
| --- |
| **PCT/CN2019/130274** |

| A. CLASSIFICATION OF SUBJECT MATTER |
| --- |
| A61B 8/08(2006.01)i |
| According to International Patent Classification (IPC) or to both national classification and IPC |

| B. FIELDS SEARCHED |
| --- |
| Minimum documentation searched (classification system followed by classification symbols) |
| A61B |
| Documentation searched other than minimum documentation to the extent that such documents are included in the fields searched |
| Electronic data base consulted during the international search (name of data base and, where practicable, search terms used) |
| CNPAT, WPI, EPODOC, CNKI: 无锡祥生医疗科技股份有限公司, 邵人杰, 赵明昌, 甘从贵, 超声, 心动周期, 切面, 收缩, 舒张, 扩张, 模型, 建模, 曲线, 射血分数, systol+, diastol+, ultraso+, cardiac+, heart, cycle, model+, curve |

| C. DOCUMENTS CONSIDERED TO BE RELEVANT | | |
| --- | --- | --- |
| Category* | Citation of document, with indication, where appropriate, of the relevant passages | Relevant to claim No. |
| X | WO 2017206023 A1 (SHENZHEN MINDRAY BIO-MEDICAL ELECTRONICS CO., LTD.) 07 December 2017 (2017-12-07) description page 4 bottom line to page 12 bottom line, figures 1-4 | 1-3, 12-13 |
| A | CN 104619259 A (BOSTON SCIENTIFIC CORPORATION) 13 May 2015 (2015-05-13) entire document | 1-13 |
| A | CN 110009640 A (TENCENT TECHNOLOGY SHENZHEN CO., LTD.) 12 July 2019 (2019-07-12) entire document | 1-13 |
| A | CN 109925002 A (HU, Qiuming et al.) 25 June 2019 (2019-06-25) entire document | 1-13 |
| A | EP 1875867 A1 (ALOKA CO., LTD.) 09 January 2008 (2008-01-09) entire document | 1-13 |
| A | US 2002072670 A1 (CHENAL, Cedric et al.) 13 June 2002 (2002-06-13) entire document | 1-13 |
| A | US 2015005629 A1 (VANDERBILT UNIVERSITY) 01 January 2015 (2015-01-01) entire document | 1-13 |

☐ Further documents are listed in the continuation of Box C.    ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **05 July 2020** | **29 July 2020** |

| Name and mailing address of the ISA/CN | Authorized officer |
| --- | --- |
| **China National Intellectual Property Administration (ISA/ CN)** **No. 6, Xitucheng Road, Jimenqiao Haidian District, Beijing 100088 China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2019/130274**

| Patent document cited in search report | | | Publication date (day/month/year) | Patent family member(s) | | | Publication date (day/month/year) |
|---|---|---|---|---|---|---|---|
| WO | 2017206023 | A1 | 07 December 2017 | CN | 108882917 | A | 23 November 2018 |
| CN | 104619259 | A | 13 May 2015 | EP | 2892433 | A1 | 15 July 2015 |
| | | | | US | 2014066764 | A1 | 06 March 2014 |
| | | | | WO | 2014039589 | A1 | 13 March 2014 |
| CN | 110009640 | A | 12 July 2019 | | None | | |
| CN | 109925002 | A | 25 June 2019 | | None | | |
| EP | 1875867 | A1 | 09 January 2008 | EP | 1875867 | B1 | 17 August 2011 |
| | | | | JP | 4206107 | B2 | 07 January 2009 |
| | | | | JP | 2008012047 | A | 24 January 2008 |
| | | | | US | 2008009735 | A1 | 10 January 2008 |
| | | | | US | 7981037 | B2 | 19 July 2011 |
| US | 2002072670 | A1 | 13 June 2002 | WO | 0245586 | A1 | 13 June 2002 |
| | | | | JP | 2004514526 | A | 20 May 2004 |
| | | | | DE | 60133044 | T2 | 26 February 2009 |
| | | | | EP | 1349499 | A1 | 08 October 2003 |
| | | | | US | 6447454 | B1 | 10 September 2002 |
| | | | | AT | 387144 | T | 15 March 2008 |
| | | | | DE | 60133044 | D1 | 10 April 2008 |
| | | | | EP | 1349499 | B1 | 27 February 2008 |
| | | | | JP | 4152746 | B2 | 17 September 2008 |
| US | 2015005629 | A1 | 01 January 2015 | US | 9883850 | B2 | 06 February 2018 |

Form PCT/ISA/210 (patent family annex) (January 2015)